(19)

**Europäisches Patentamt**
**European Patent Office**
**Office européen des brevets**

(11) **EP 2 803 317 B1**

(12) **EUROPEAN PATENT SPECIFICATION**

(45) Date of publication and mention
of the grant of the patent:
**09.12.2020 Bulletin 2020/50**

(51) Int Cl.:
*G01R 33/563* (2006.01)     *G01R 33/56* (2006.01)
*G01R 33/58* (2006.01)     *A61B 5/055* (2006.01)

(21) Application number: **12864813.6**

(22) Date of filing: **13.01.2012**

(86) International application number:
**PCT/CN2012/070352**

(87) International publication number:
**WO 2013/104132 (18.07.2013 Gazette 2013/29)**

(54) **MRI METHOD FOR MEASURING DIFFUSION PARAMETERS OF TRACER IN LIVING ORGAN TISSUE SPACE AND EXTRACELLULAR SPACE**

MRT-VERFAHREN ZUR MESSUNG VON DIFFUSIONSPARAMETERN EINES TRACERS IN LEBENDEM ORGANISCHEM GEWEBE UND IN EINEM EXTRAZELLULÄREN RAUM

PROCÉDÉ IRM POUR LA MESURE DE PARAMÈTRES DE DIFFUSION D'UN TRACEUR DANS UN ESPACE TISSULAIRE ET UN ESPACE EXTRACELLULAIRE D'ORGANES VIVANTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**19.11.2014 Bulletin 2014/47**

(73) Proprietor: **Peking University Third Hospital Haidian District, Beijing 100091 (CN)**

(72) Inventor: **HAN, Hongbin**
**Beijing 100091 (CN)**

(74) Representative: **Gunzelmann, Rainer**
**Wuesthoff & Wuesthoff**
**Patentanwälte PartG mbB**
**Schweigerstraße 2**
**81541 München (DE)**

(56) References cited:
**WO-A1-2011/069283     WO-A2-02/49512**
**CN-A- 102 238 908     CN-A- 102 293 634**

• **PAUL S TOFTS: "Modeling Tracer Kinetics in Dynamic Gd-DTPA MR Imaging", JOURNAL OF MAGNETIC RESONANCE IMAGING, vol. 7, no. 1, 1 January 1997 (1997-01-01) , pages 91-101, XP055322552, DOI: doi:10.1002/jmri.1880070113**

• **J P B O'CONNOR ET AL: "Dynamic contrast-enhanced imaging techniques: CT and MRI", BRITISH JOURNAL OF RADIOLOGY., vol. 84, no. special_issue_2, 1 December 2011 (2011-12-01), pages S112-S120, XP055322599, GB ISSN: 0007-1285, DOI: 10.1259/bjr/55166688**

• **MATTHIAS C SCHABEL ET AL: "Uncertainty and bias in contrast concentration measurements using spoiled gradient echo pulse sequences; Uncertainty and bias in SPGR concentration measurements", PHYSICS IN MEDICINE AND BIOLOGY, INSTITUTE OF PHYSICS PUBLISHING, BRISTOL GB, vol. 53, no. 9, 17 April 2008 (2008-04-17) , pages 2345-2373, XP020133975, ISSN: 0031-9155**

• **PELLERIN M ET AL: "Incorporating Contrast Agent Diffusion Into the Analysis of DCE-MRI Data", MAGNETIC RESONANCE IN MEDICINE, JOHN WILEY & SONS, INC, US, vol. 58, 29 October 2007 (2007-10-29), pages 1124-1134, XP002507059, ISSN: 0740-3194, DOI: 10.1002/MRM.21400 [retrieved on 2007-10-29]**

**(Cont. next page)**

- TOFTS P S ET AL: "ESTIMATING KINETIC PARAMETERS FROM DYNAMIC CONTRAST-ENHANCED T1-WEIGHTED MRI OF A DIFFUSABLE TRACER: STANDARDIZED QUANTITIES AND SYMBOLS", JOURNAL OF MAGNETIC RESONANCE IMAGING, SOCIETY FOR MAGNETIC RESONANCE IMAGING, OAK BROOK, IL, US, vol. 10, no. 3, 1 September 1999 (1999-09-01), pages 223-232, XP001118038, ISSN: 1053-1807, DOI: 10.1002/(SICI)1522-2586(199909)10:3<223::AID-JMRI2>3.0.CO;2-S
- C. NICHOLSON: "Factors governing diffusing molecular signals in brain extracellular space", JOURNAL OF NEURAL TRANSMISSION, vol. 112, no. 1, 14 September 2004 (2004-09-14), pages 29-44, XP055077479, ISSN: 0300-9564, DOI: 10.1007/s00702-004-0204-1
- FANGJINGWEI XU ET AL: "Quantification of Gd-DTPA concentration in neuroimaging using T3D MP-RAGE sequence at 3.0 T", MAGNETIC RESONANCE IMAGING, ELSEVIER SCIENCE, TARRYTOWN, NY, US, vol. 29, no. 6, 20 February 2011 (2011-02-20), pages 827-834, XP028378890, ISSN: 0730-725X, DOI: 10.1016/J.MRI.2011.02.019 [retrieved on 2011-03-28]
- E. SYKOVA ET AL: "Diffusion in Brain Extracellular Space", PHYSIOLOGICAL REVIEWS, vol. 88, no. 4, 1 October 2008 (2008-10-01), pages 1277-1340, XP055077486, ISSN: 0031-9333, DOI: 10.1152/physrev.00027.2007
- FANGJINGWEI XU ET AL: 'Quantification of Gd-DTPA concentration in neuroimaging using T1 3D MP-RAGE sequence at 3.0 T' MAGNETIC RESONANCE IMAGING vol. 29, no. ISS.6, July 2011, pages 827 - 834, XP028378890
- C.NICHOLSON.: 'Factors governing diffusing molecular signals in brain extracellular space' JOURNAL OF NEURAL TRANSMISSION vol. 112, January 2005, pages 29 - 44, XP055077479
- XU, FANGJINGWEI ET AL.: 'Measurement of brain extracellular space and its physiological and Pathophysiological significance' JOURNAL OF PEKING UNIVERSITY (HEALTH SCIENCES) vol. 42, no. 2, April 2010, pages 234 - 237, XP055077482
- HE, QINGYUAN ET AL.: 'Imaging and quantitative measurement of brain extracellular space using MRI Gd-DTPA tracer method' JOURNAL OF PEKING UNIVERSITY (HEALTH SCIENCES) vol. 42, no. 2, April 2010, pages 188 - 191, XP055077485
- EVA SYKOVA ET AL.: 'Diffusion in Brain Extracellular Space' PHYSIOL REV. vol. 88, no. 4, October 2008, pages 1277 - 1340, XP055077486
- MAES F ET AL: "Medical image registration using mutual information", PROCEEDINGS OF THE IEEE, IEEE. NEW YORK, US, vol. 91, no. 10, 1 October 2003 (2003-10-01), pages 1699-1722, XP011100853, ISSN: 0018-9219, DOI: 10.1109/JPROC.2003.817864
- JI ET AL: "A novel, fast entropy-minimization algorithm for bias field correction in MR images", MAGNETIC RESONANCE IMA, ELSEVIER SCIENCE, TARRYTOWN, NY, US, vol. 25, no. 2, 30 January 2007 (2007-01-30), pages 259-264, XP005867329, ISSN: 0730-725X, DOI: 10.1016/J.MRI.2006.09.012

**Description**

**Field of the Invention**

The invention relates to a method for computing the parameter of a tracer distributing in the living organism and diffusing in the ECS (Brain Extracellular Space), especially to a method for analyzing and computing the diffusion parameters of the tracer in the living organism with the aid of the MRI image.

**Background of the Invention**

In the pharmacokinetics study, in order to evaluate the drug diffusion and predict its effect in target, it needs to execute and dissect animals and get their tissue samples or collect venous blood from healthy volunteers so as to measure the drug concentrations of the tissue and the blood from different time points after administration. Using this traditional research method, the subject will be invaded and only macro-scopic distribution rules of the drug can be obtained. The micro distribution rules of the drug are still unavailable. In addition, the reserving and transferring the samples will cost much before in-vitro measurement.

With the rapid development of the radiological technology, the radioactive tracer method provides the possibility of in-vivo study and visual measurement for drug distribution and clearance process. However, iodine and the other tracers depend on the X-ray, CT and other imaging equipment. The X-ray, CT and the radioactive isotope can generate ionizing radiation and ionization damage which are harmful to the subject. The MRI (Magnetic Resonance Imaging) technique has become a widely-used imaging technique. Using MRI to observe the anatomical structure and physiological function of human body or animal has the advantages of in-vivo, non-invasive and real-time imaging. The MRI tracer further develops the application of MRI technique.

At present, two kinds of tracer are primarily used in the MRI examination: one is T1 positive tracer represented by Gadolinium-diethylenetriaminepentaacetic acid (Gd-DTPA) and the other is T2 negative tracer represented by iron nanoparticle. The pilot study has been conducted by the academics on clearance route for metabolic substance in the ISF (interstitial fluid) via using the iron nanoparticle as the MRI tracer. The study confirmed that the iron nanoparticle injected in the brain is cleared out from nasal mucosa lymphoid tissue to cervical lymph nodes.

However, the research also shows that the tracer in the living organism are difficult to be observed and quantitively analyzed owing to the image deformation and signal loss caused by the interference of the iron nanoparticle on the gradient field.

The DWI (Diffusion Weighted Imaging) has also been used in measurement of the living organism. The DWI is a MRI technique used for measuring ADC (Apparent diffusion Coefficient) of the water and other molecules in body and FA (Anisotropy Fraction) of the tissue. This technique is based on the principle of molecular diffusion change causing MRI image signal change in tissue. When applying a lining diffusion sensitive gradient field on a certain direction, the MRI signal intensity is low where diffusion process along said direction inside the tissue is obvious, and vise versa. The ADC values can be calculated via different MRI signal intensities caused by different diffusion sensitive gradients. By applying more than six diffusion sensitive gradients with different directions, the parameters of diffusion tensor can be obtained, such as the FA, which is ratio of the anisotropy to the whole diffusion and reflects the movement intensity of the molecules on the main vector shaft of the diffusion.

Therefore, developing a method for quantitively measuring the diffusion parameters in vivo has great value on the studying of the metabolism and clinical drug treatment at the particular anatomic site.

The Chinese patent application CN2010102086244 relates to a method for measuring physiological parameters of ISF and ECS. Using magnetic relaxation property of the micromolecule tracer Gd-DTPA to get the distribution and clearance process in three-dimensional space of interstitial brain acquired by MIR, this technique obtains anatomical and physiological parameters of ECS via inversion algorithm of classical diffusion equation. However, the quantitative analysis of the spatial and temporal distribution of the tracer in the brain ECS is not involved in this patent.

Document M. Pellerin, "Incorporating Contrast Agents Diffusion Into the Analysis of DCE-MRI Data", Magnetic Resonance in Medicine 58, p. 1124-1134, 2007, describes a diffusion-perfusion model in which diffusion of a low molecular weight contrast agent is incorporated in a standard two-compartment Tofts model.

WO 2011/069283 A1 concerns a method for measuring physiological parameters in cerebral interstitial fluid (ISF) and cerebral extracellular space (ECS). It is a method for measuring physiological parameters such as diffusion, flow and elimination etc. of materials among the cerebral ISF within the cerebral ECS. The method comprises the steps of: placing the subject's brain in the magnetic resonance imaging device, administering a magnetic resonance imaging contrast agent into the subject's cerebral ISF, afterwards measuring the signal intensity of the contrast agent in MRI image, and according to the signal intensity, determining the distribution of the contrast agent at the corresponding part, and the concentration and concentration change rate of the contrast agent in each brain region.

**Summary of the Invention**

The present invention provides a method for computing the parameter of tracer distributing in the living organism and diffusing in the ECS according to claim 1. Further aspects of the present invention are defined in the dependent claims. The present invention obtains the diffusion feature of the tracer in the living organism via the MRI signal change caused by the diffusing of the tracer in the living organism so that the distribution change and clearance process of the tracer caused by the diffusion in the living organism can be directly reflected.

Furthermore, the present invention segments and registers the acquired three-dimensional image with the aid of MRI tracing technique, then uses anisotropic diffusion equation to finally get the spatial structure parameter of the living organism and the diffusion and clearance parameter of the tracer therein, as well as provides the pharmacokinetic parameter including the volume distribution of the molecule the and the overall clearance. Certain exemplary embodiments can provide a method, in which the tracer is Gd-DTPA and the living organism is brain ECS.

The present invention provides a method for computing the parameter of tracer distributing in the living organism and diffusing in the ECS, comprising: (1)measuring magnetic resonance signal increment (∆SI) corresponding to the different tracer concentrations (C) in agarose; (2)according to the value measured in the step (1), determining a upper limit of the tracer concentration when having a linear relation with the magnetic resonance signal increment (∆SI) and determining the linear relation between the magnetic resonance signal increment (∆SI) and the tracer concentration (C); (3) obtaining magnetic resonance images from different times of the living organism, who has been injected the tracer with different concentrations of tracer at injection sites (Q);(4) orderly registering the regions of interest from the obtained magnetic resonance images; and (5) fitting the parameter of the tracer distributing in the living organism according to the registered images, which comprises: (5.1) determining the diffusion equation of the tracer, (5.2) conversing the equation determined in the step (5.1) according to the relation between the tracer concentration and the magnetic resonance signal increment, (5.3) solving the equation acquired in the step (5.2) to fit the diffusion parameter including diffusion coefficient D*, loss ratio k' and /or part volume ratio a of the tracer.

Certain examples outside the scope of the present invention and useful for understanding the invention can provide a method, in which the diffusion equation of the tracer diffusing from the injection site (Q) to a certain point (P) in the space determined in the steps (5.1) is:

$$\frac{\partial C}{\partial t} = \frac{D}{\lambda_{PQ}^2} \frac{\partial^2 C}{\partial r^2} + \frac{Q}{\alpha} - k'C$$

wherein, D is a free diffusion coefficient of the tracer ;r is the distance from the calculation point (P) to the injection site (Q) of the tracer; C is the tracer concentration which is obtained via the magnetic resonance signal (∆SI);t is a time interval between the tracer injection and the magnetic resonance image acquisition; a is a ratio of the volume of the brain ECS to the whole volume of the animal tissue; k' is a the clearance rate of the tracer in the living organism which is a constant; $\lambda_{PQ}$ is a tortuosity between the calculation point (P) and the tracer injection site (Q) in the living organism, which reflects the barrier from the structure of the living organism to the molecular diffusion, $\lambda_{PQ}=\lambda(q, j)$, q and j are the angles between the connecting line from tracer injection site (Q) to the calculation point (P) and axis X,Z. The equation conversed in the Step (5.2) is:

$$\begin{cases} \dfrac{\partial(\Delta SI)}{\partial t} = \dfrac{D}{\lambda_{PQ}^2} \dfrac{\partial^2(\Delta SI)}{\partial r^2} + \dfrac{Q}{\alpha}k - k'(\Delta SI) + k'b \\ \Delta SI(r,0) = \begin{cases} \dfrac{Qk}{\alpha} + b, & -0.5d < r < 0.5d \\ 0, & \text{others} \end{cases} \end{cases}$$

wherein, K and B are constants, ∆SI is value of magnetic resonance signal intensity, d is the real space distance corresponding to a single pixel; solving the above equation in the Step (5.3) can obtain the root as:

$$\Delta SI = \frac{\lambda_{PQ}}{2\pi\sqrt{Dt}} \int_{-\infty}^{+\infty} \phi(\xi) e^{\left(-\lambda_{PQ}^2 \frac{(r-\xi)^2}{4Dt} - k't\right)} d\xi + \left(\frac{Qk}{\alpha k'} + b\right)\left(1 - e^{-k't}\right)$$

and wherein, the $\pi$ and e are the mathematical constants, $\xi$ is the real space distance corresponding to a single pixel in the magnetic resonance image.

In the method provided by the present invention, the diffusion equation of the tracer in the standard coordinates obtained in the step (5.1) is:

$$\frac{\partial C}{\partial t} = \frac{D}{\lambda(x, y, z)^2} \nabla^2 C + \frac{Q}{\alpha} - k'C ,$$

wherein, D is the free diffusion coefficient of the tracer, $\lambda$ (x, y, z) is the tortuosity of a point(x, y, z) in the living organism, C is the tracer concentration which is obtained via the magnetic resonance signal increment ($\Delta$SI), t is the time interval between the tracer injection and the magnetic resonance image acquisition, a is the ratio of the volume of brain ECS to all volume of the living organism, k' is the clearance rate of the tracer in the living organism which is a constant. The diffusion equation in a anisotropic condition can be equivalently transformed into:

$$\frac{\partial C}{\partial t} = \frac{D}{\lambda_x^2}\frac{\partial^2 C}{\partial x^2} + \frac{D}{\lambda_y^2}\frac{\partial^2 C}{\partial y^2} + \frac{D}{\lambda_z^2}\frac{\partial^2 C}{\partial z^2} + \frac{Q}{\alpha} - k'C ,$$

wherein, D is the free diffusion coefficient of the tracer; $\lambda_x$, $\lambda_y$ and $\lambda_z$ are the tortuosities of the living organism along the axial X, Y and Z respectively; C is the tracer concentration which is obtained via the magnetic resonance signal increment ($\Delta$SI); t is the time interval between the tracer injection and the magnetic resonance image acquisition; a is the ratio of volume of the brain ECS to all volume of the living organism; k' is the clearance rate of the tracer in the living organism which is a constant.

The equation after converting in the step (5.2) is:

$$\frac{\partial(\Delta SI)}{\partial t} = \frac{D}{\lambda(x,y,z)^2}\frac{\partial^2(\Delta SI)}{\partial r^2} + \frac{Q}{\alpha}k - k'(\Delta SI) + k'b ,$$

wherein, k and b are constants, $\Delta$SI is the magnetic resonance signal increment, d is the real space distance corresponding to a single pixel. The above equation is conversed into a discrete model in the step (5.3) as follows:

$$\Delta SI(t+\Delta t) - \Delta SI(t) = \frac{D}{\lambda(x,y,z)^2}\nabla^2(\Delta SI) + \frac{Q}{\alpha}k - k'\Delta SI(t) + k'b ,$$

Wherein, $\Delta$t is the sampling time in unit; $\lambda$ (x, y, z) is the tortuosity of a point(x, y, z) in space, $\Delta$i is the space distance of the sample in any direction (axis i).

In the method provided by the present invention, the equation obtained in the step (5.3) can be fitted via the least square method using the following equation:

$$\min f(\lambda,\alpha,k') = \sum_x\sum_y\sum_z\sum_t\left(\frac{D}{\lambda(x,y,z)^2}\frac{\partial^2(\Delta SI)}{\partial r^2} + \frac{Q}{\alpha}k - k'(\Delta SI) + k'b - \Delta SI(t+\Delta t) + \Delta SI(t)\right)^2 ,$$

wherein, mín $f(\lambda,\alpha,k')$ is the minimum value of the variance. The above formula is expressed in matrix form as: mín $f(\lambda,\alpha,k')=\|Ax-b\|^2$. The non-zero element in the row (x, y, z, t,:) in the matrix A is: $b(x,y,z,t,:)=\Delta SI(t+\Delta t)-\Delta SI(t)$. The

row (x, y, z, t,:) of known vector b is: $b(x,y,z,t,:) = \Delta SI(t + \Delta t) - \Delta SI(t)$. The substitution variable x is:

$$x = \left[ \frac{D}{\lambda(x,y,z)^2}, \frac{Q}{\alpha}, k' \right]^T.$$

Certain exemplary embodiments can provide a method, in which the image registration steps comprises:

$$I(f,g) = H(f) + H(g) - H(f,g)$$
$$= -\sum_{i=1}^{N} p(i)\log(p(i)) - \sum_{j=1}^{M} p(j)\log(p(j)) + \sum_{i=1}^{N}\sum_{j=1}^{M} p(i,j)\log(p(i))$$
$$= \sum_{i=1}^{N}\sum_{j=1}^{M} p(i,j)\log(\frac{p(i,j)}{p(i)p(j)}),$$

wherein, $H(f)$, H(g) and $H(f,g)$ are the edge entropy and joint entropy of the two images, N and M are the numbers of grayscale samples of the two images, p(i), p(j) and p(i, j) are the possibilities of the grayscale appearing.

Certain exemplary embodiments can provide a method, in which the registration process of the step (4) further comprises a grayscale correction as following:

$$I(x,y,z) = f_{MR}(x,y,z)B(x,y,z) + n(x,y,z),$$

wherein, $f_{MR}$ is the real NMR signal not polluted by noise and bias field, I is the imaging signal, B is the grayscale bias field, n is the artificial noise introduced in the imaging process.

Certain exemplary embodiments can provide a method, in which the grayscale correction further comprises:

$$g' = u_2 + (g - u_1) \times \frac{\sigma_2}{\sigma_1},$$

wherein, $(u_1, \sigma_1)$ and $(u_2, \sigma_2)$ are the mean values and the variances of the magnetic resonance signal intensities in two areas to be corrected, g is the real MRI image signal intensity, g' is the corrected MRI image signal intensity.

Certain exemplary embodiments can provide a method, in which the tracer is Gd-DTPA, the diffusion parameters of the living organisms comprises the effective diffusion efficient D*, tortuosity $\lambda$ and clearance rate k' of Gd-DTPA in the ECS.

Using the method according to the present invention, the diffusion parameters reflecting the structure and physical feature of the tissue cell, as well as the distribution and clearance process of the tracer in brain are both can be obtained in living organism. The present method can calculate the micro parameter such as the effective diffusion efficient D*, the tortuosity $\lambda$ and the clearance rate k' as well as the macro parameter such as distribution volume (Vd) and distribution volume fraction, so that can design the disease treatment protocol and be helpful to the clinical medication. For example, valuable data for analysis can be acquired by calculating the diffusion and clearance data of Gd-DTPA in the brain ECS.

**Brief Description of the Drawings**

FIG. 1 shows MRI images with different Gd-DTPA concentrations in MRI device.
FIG. 2 shows the relation between the Gd-DTPA concentration (C) and the MRI signal intensity increment ($\Delta$SI) when the Gd-DTPA concentration ranging from 0-100mM.
FIG. 3 shows the relation between the Gd-DTPA concentration (C) and the MRI signal intensity increment ($\Delta$SI) when the Gd-DTPA concentration ranging from 0-4mM.
FIG. 4 shows under wrist coil imaging, the linear relation between the Gd-DTPA concentration (C) and the MRI signal intensity increment (ΔSI) when the Gd-DTPA concentration ranging from 0-1.5mM.

**FIG. 5** shows under head coil imaging, the linear relation between the Gd-DTPA concentration (C) 0-1.5mM and the MRI signal intensity increment (ΔSI) when the Gd-DTPA concentration ranging from 0-1.5mM.

**FIG. 6** schematically shows the method for processing the above-acquired time-series MRI images.

**FIG. 7** shows the obvious deformation in most tissue caused by movement of the rat during imaging.

**FIG. 8** shows the registration framework of the time-weighted MRI images according to the present invention.

**FIG. 9** shows using Matlab software to measure the un-increment region subtracting increment region.

**FIG. 10** shows the graphical results of measuring the MRI increment signal of the increment region in FIG. 9, wherein the axis X is signal intensity and the axis Y is the number of pixels.

**FIG. 11** shows the images of SD rats scanned from different times after injection of Gd-DTPA (2μL, 10mM) via the injection sites of (a) the caudate nucleus, (b) substantia nigros, (c) thalamus, (d) occipital cortex and (e) subcortical white matter.

**FIG. 12** shows the diffusion MRI images scanned from different times after injecting the Gd-DTPA into the rat skeletal muscle.

**FIG. 13** shows the diffusion images of Gd-DTPA in the glioma models of the rat brains, wherein (a) the high signal area of the T1WI is diffusion areas of Gd-DTPA and the low signal area is the gliomas location, (b) high signal area of the T2WI is the gliomas location.

## Detailed Description of the Embodiments

The preferred embodiments of the present invention are described below with reference to the accompanying drawings.

A method for computing the physiological parameter in the living organism will be illustrated with the following embodiment. In the embodiment, Gadolinium-diethylenetriaminepentaacetic acid (Gd-DTPA) which is Mag-nevist solution produced by Bayer Schering Pharma AG was used as the tracer. The corresponding MRI signal intensities of the tracer with the actual concentration in agarose gel and rat brain were measured. Male Sprague Dawley rats weighing 250-300g were used in the experiment. The living organism used for measurement is the rat brain. A Siemens Magnetom Trio 3.0 T MRI system was used to obtain the rat brain images.

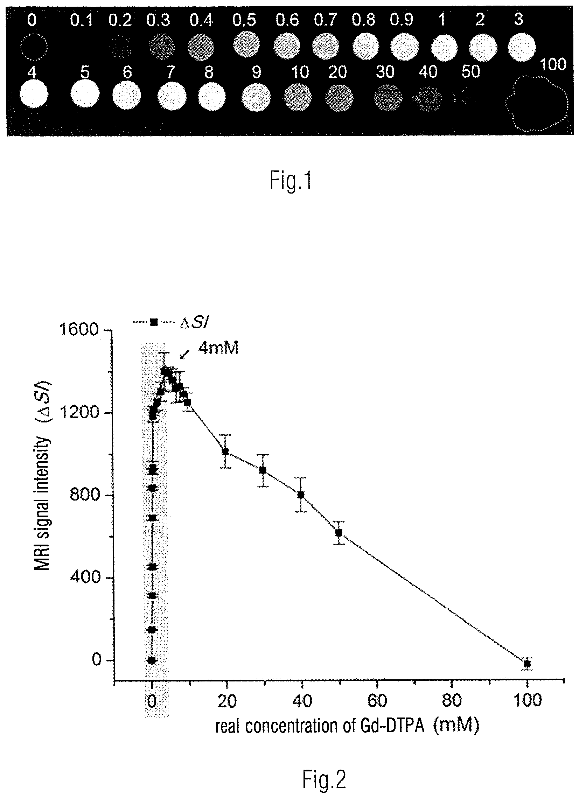

**FIG. 1** shows MRI images of with different Gd-DTPA concentrations in MRI device, wherein shows MRI images of the agarose gel obtained via an eight channels wrist coil after injecting different concentrations of Gd-DTPA ranging from 0-100mM at 37°C. A 3D MP-RAGE (Magnetization Prepared Rapid Gradient Echo) was used for T1-weighted sequence. The parameter was as follows: ET (echo time) = 3.7 ms, RT (repetition time) = 1500 ms, flip angle =9°, TI (inversion time) = 900 ms, FOV (field of view) = 267 mm, pixel = $0.5 \times 0.5 \times 0.5$ mm3, matrix = $512 \times 96$.

**FIG. 2** shows the relation between the Gd-DTPA concentration (C) and the MRI signal intensity increment (ΔSI) when the Gd-DTPA concentration (C) ranging from 0-100mM. The result shows that: when the concentration ranging from 0-100mM, as concentration of Gd-DTPA (C) increases, the MRI signal intensity increment (ΔSI) increases first then decreases. The curve is bell-shaped and reaches its peak at 4mM then gradually declining. The artifact appeared at 50mM. The value of ΔSI turns into negative when the Gd-DTPA concentration becomes 100mM, indicating that the MRI signal intensity has been lower than that of pure water while the Gd-DTPA concentration becomes 100mM.

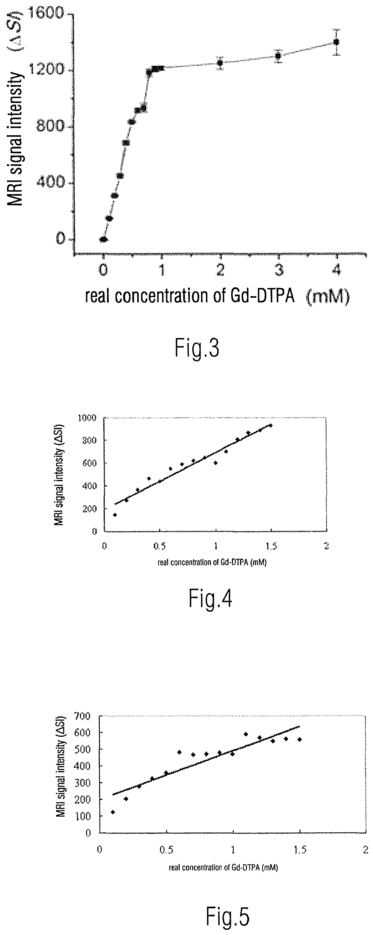

**FIG. 3** shows the relation between the Gd-DTPA concentration (C) and the MRI signal intensity increment (ΔSI) when the Gd-DTPA is ranging from 0-4mM. When the Gd-DTPA concentration (C) ranging from 0-1mM, the MRI signal intensity increment (ΔSI) rapidly linear-increases with the rise of the concentration (C) and reaches its peak at 4mM. Thus, it is concluded that when the MRI signal intensity increment (ΔSI) and the concentration (C) are in a linear relationship, the upper limit of the tracer concentration is 4mM.

**FIG. 4** shows under wrist coil imaging, the linear relation between the signal intensity increment (ΔSI) of the relating Gd-DTPA and the Gd-DTPA concentration (C) when the Gd-DTPA concentration ranging from 0-1.5mM, wherein, the fitting linear equation is: y=499.54x+194.76,
the fitting goodness is: $R^2$=0.9538.

**FIG. 5** shows under head coil imaging, the linear relation between the signal intensity increment (ΔSI) of the relating Gd-DTPA and Gd-DTPA concentration (C) when the Gd-DTPA concentration ranging from 0-1.5mM, wherein, the fitting linear equation is: y=294.26x+197.9,
the fitting goodness :$R^2$=0.8379.

Thus it can be seen that linear relation between the Gd-DTPA concentration and the corresponding signal intensity increment ΔSI varies while using different imaging coils. To get a good fitting curve, we can choose different RF coils according to different organs of the living body when imaging.

In this embodiment, the position of the injection site Q and the depth of Gd-DTPA microinjection of the rat brains were determined by three-dimensional reconstructed image basing on the brain stereotaxic atlas. The specific method was: preparing skin, shaving between the eyes of rats about 4cm skin backwards along the midline of the skull and exposing the skull plate; then fixing the rats in a stereotactic apparatus and administrating the drug at the injection site Q using magnetic drug delivery system; rubbing off the skull plate at the entry point with an electric drill, setting a micro syringe into the brain and manually administrating 2μLGd-DTPA solution with the concentration of 10mM into the target location in three injection with the interval of 5min. The syringe was left for 10 min in the brain when drug injection was finished before the micro syringe was exited and the skin was sutured. Scanned images were taken in 15min, 30min, 1h, 2h, 3h, 4h, 6h, 9h and 12h and imaging parameters were set as the mentioned above.

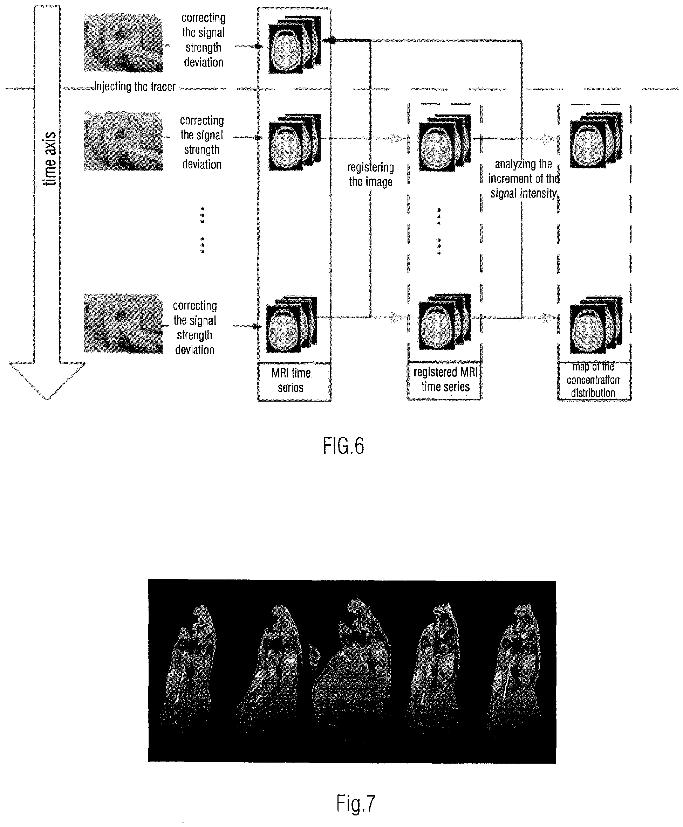

**FIG. 6** schematically shows the method for processing the above acquired time-series MRI images. As shown in the FIG., the bias field is first to be correct to eliminate the signal strength deviation among the sequence of images; the image registration technique is then used to unify the position of the increment images with the referenced images; the diffusion model is finally solved via the distribution of the tracer concentration from different times obtained by the analysis of the signal strength increment.

Since it takes a long time to acquire the MR image, the position of measured object will inevitably change during acquisition process. Therefore, before the subtraction of the images, the time series are aligned with the same coordinate system via image registration techniques. Besides, the grayscale of the MRI image are uneven because of the impact of bias field due to the imperfections of the MRI procedure, i.e. the grayscale will smoothly change in the imaging area of a same even tissue. If there is no correction of the bias field, the subtracted difference of grayscale does not reflect the tracer concentration even though two images are aligned with the reference position because the grayscale of pixel changes according to the different imaging position in bias field. Thus, the accuracy validation is assured and the diffusion model is solved via registration and uneven grayscale correction of the images having character of MR time series.

The tracer diffusion process is embodied in MR time series image. The grayscale distribution of images from different times varies, which makes difficulty to the similarity measurement. In addition, the rigid transformation and the elastic deformation may both exist in image, which affect the time-series MR image registration, e.g. as shown in **FIG. 7,** in the imaging process, the significant deformation in the most tissue was caused by the movement of rat, but the rigid transformation only exist in the brain.

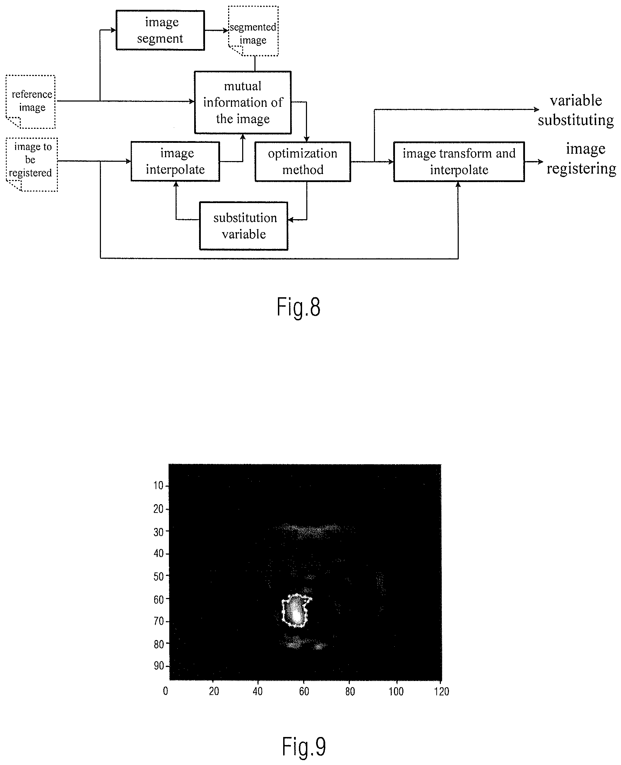

In an exemplary embodiment of the present invention, the image registration method in combination with the ROI (region of interest) segmentation is used and the mutual information is selected as the similarity criterion. **FIG. 8** exemplary shows the framework of the algorithm.

The mutual information is a criterion of similarity basing on information theory and is widely used in registration of the multi and the single mode images. Regarding the two images (f and g) to be registered, the mutual information is defined as the difference between edge entropy and joint entropy of the two images:

$$
\begin{aligned}
I(f,g) &= H(f) + H(g) - H(f,g) \\
&= -\sum_{i=1}^{N} p(i)\log(p(i)) - \sum_{j=1}^{M} p(j)\log(p(j)) + \sum_{i=1}^{N}\sum_{j=1}^{M} p(i,j)\log(p(i)) \\
&= \sum_{i=1}^{N}\sum_{j=1}^{M} p(i,j)\log\left(\frac{p(i,j)}{p(i)\,p(j)}\right)
\end{aligned}
\tag{1},
$$

wherein, H(*f*), H(g) and H(*f*,g) are the edge entropy and joint entropy of two images; N and M are the number of samples of two grayscale value in the two images; p(i), p(j) and p (i, j) are the probability of grayscale value appears. The mutual information is a kind of a regional grayscale statistical measurement and does not require the grayscale correspondence between points of two images. Therefore, it has a high robustness of the image noise and the grayscale change. The mutual information has more advantages in similarity measurement comparing with other methods especially for MR time-series images due to the grayscale inhomogeneity and the grayscale change caused by the tracer.

In addition, as shown in **FIG. 8,** using image segmentation module in the framework in the image registration, the calculation of the similarity measurement of the mutual information is limit within the region of interest (such as the brain of rats), so that only the rigid transformation is considered in the registration process. Thus the influence of the elastic deformation of the surrounding tissue to the registration accuracy is avoided. Furthermore, for fast and accurately extracting of the region of interest, the interactive image segmentation method basing on the graph theory is used to solve the problem of the lost of the part edge of the target, which is caused by noise disturbance and the lack of grayscale contrast. The method supports the rapid amendment basing on the initial segmentation result. Therefore, it is suitable for the extraction of region of interest under complicated situation of MR time series images to improve the successful rate of image registration.

In an exemplary embodiment of the present invention, regarding the MR signal intensity change for the time-series images, the image correction method is: firstly correcting the bias field for each set of image, then unifying the signal intensity among sequences.

According to the most commonly used model of MRI, the imaged MR signal can be represented as:

$$I(x, y, z) = f_{MR}(x, y, z)B(x, y, z) + n(x, y, z) \quad (2),$$

wherein, I is the imaging signal;

$f_{MR}$ is the real NMR signal which is not polluted by noise and non-uniform field;

B is the grayscale bias field;

n is the additive noise introduced during the imaging.

Thus, the bias field can be modified by estimating the grayscale bias field B, so that the real MR signal can be recovered using the formula (2).

To solve this problem, we intend to use correction method basing on High-Frequency Maximization. The distribution of bias field is obtained via an iteration process of the distribution of the signal intensity of the images in order to maximize the high-frequency content of the grayscale distribution of the images. This method does not require prior knowledge and can be automatic performed, therefore, is more suitable for processing the MR image which has a large data.

The bias field correction can improve the consistency of the signal intensity distribution within a single set of images. However, the consistence of grayscale distribution of the same tissue among the series may be reduced after correction because only intensity information within a single set of images is taken for consideration. This problem is more obvious especially when the signal intensity drifts. Therefore, in an exemplary embodiment of the present invention, a correction method using grayscale value statistical characteristic to uniform the signal intensity is further included.

Given two images $f_1$ and $f_2$ for correction, two regions of interest, $R_1$ and $R_2$ are picked from the same positions in the imaging regions from the uniform tissue. The $(u_1, \sigma_1)$ and $(u_2, \sigma_2)$ are get via accounting the mean values and variances of the signal intensities in the regions of interest. The grayscale uniform correction is realized by the linear transformation below:

$$g' = u_1 + (g - u_1) \times \frac{\sigma_2}{\sigma_1} \quad (3),$$

wherein, g and g' are respectively the signal intensity before and after transformation.

The ROI can be determined via human interaction or automatic selected by region clustering and the corresponding methods of region matching based on the Mean-Shift algorithm.

As shown in **FIG. 8,** segmenting regions of interest of the standard reference images and images to be registered, then registering the images via mutual information according to formula(1) or correcting the grayscale using high-frequency-maximization basing method according to formula (3), the corrected image is obtained. Using the above method, the tracer concentration in a particular region in the living organism can be obtained via the MRI signal intensity increment. Basing on the obtained tracer concentration, the present invention can establish a diffusion model of the tracer in the living organism and work out the parameter reflecting the feature of the living organism.

In examples outside the scope of the present invention and useful for understanding the present invention, for reflecting the structure of ECS, assuming that the tortuosity between a point P and the injection site Q is only depend on the direction:

$$\lambda_{PQ} = \lambda(\theta, \varphi) \quad (4),$$

wherein, $\theta$ is the intersection angle between PQ and axis x; $\varphi$ is the intersection angle between PQ and axis z.

Therefore, the diffusion equation along the direction of PQ can be:

$$\frac{\partial C}{\partial t} = \frac{D}{\lambda_{PQ}^2} \frac{\partial^2 C}{\partial r^2} + \frac{Q}{\alpha} - k'C \quad (5),$$

wherein, D is the free diffusion coefficient of the tracer in the agarose gel;

r is the distance between the calculation point P and the injection site Q;

C is the concentration of the tracer in the part of brain tissue, obtaining from calculating of the MRI signal increment (ΔSI);

t is the time interval between the tracer injection and the acquisition of MRI image;

a is the volume ratio of the brain ECS to the whole brain;

k' the clearance rate of the tracer in the brain which is a constant;

$\lambda_{PQ}$ is the tortuosity of the brain between the point P and the tracer injection site Q.

It can be seen that different from the current diffusion models, the diffusion model basing on the equation (5) is alterable in each direction so that the irregular diffusion surface is available when the tortuosity in each direction is calculated.

Due to the approximately linear relation between the tracer concentration and the MRI image signal increment, the MRI signal increment ΔSI satisfied the diffusion equation as below:

$$\begin{cases} \dfrac{\partial(\Delta SI)}{\partial t} = \dfrac{D}{\lambda_{PQ}^2}\dfrac{\partial^2(\Delta SI)}{\partial r^2} + \dfrac{Q}{\alpha}k - k'(\Delta SI) + k'b \\ \Delta SI(r,0) = \begin{cases} \dfrac{Qk}{\alpha} + b, & -0.5d < r < 0.5d \\ 0, & \text{others} \end{cases} \end{cases} \tag{6},$$

wherein, d is the real space distance relating to a single pixel.

Thus, the equation solution of formula (5) is obtained as:

$$\Delta SI = \dfrac{\lambda_{PQ}}{2\pi\sqrt{Dt}} \int_{-\infty}^{+\infty} \phi(\xi) e^{\left(-\lambda_{PQ}^2 \dfrac{(r-\xi)^2}{4Dt} - k't\right)} d\xi + \left(\dfrac{Qk}{\alpha k'} + b\right)\left(1 - e^{-k't}\right) \tag{7}.$$

The diffusion coefficient

$$D_{PQ}^* = D / \lambda_{PQ}^2 ,$$

the loss ratio k' and the part volume ratio α along the PQ direction can be fit via the acquisition of all pixel values of all the lines PQ from different times.

Although the tortuosity varies in different directions and remains unchanged in the same direction in equation (5), the real tortuosity probably varies in the same direction. The space variability of the tortuosity should be determined to accurately describe the real diffusion process. The tortuosity of a point (x, y, z) in space is:

$$\lambda = \lambda(x, y, z) \tag{8},$$

or written in a polar coordinates:

$$\lambda = \lambda(r, \theta, \varphi) \tag{9},$$

the diffusion model is changed to:

$$\frac{\partial C}{\partial t} = \frac{D}{\lambda(x, y, z)^2} \nabla^2 C - \frac{Q}{\alpha} - k'C \qquad (10).$$

Although equation (10) fully considers the space variability of the tortuosity, the solution of equation (8) is difficult to get. The equation (8) is a partial differential formula with variable coefficients and usually has no analytic solution. The continuous problem can be turned into a discrete problem to solve the equation (10):

$$\Delta SI(t + \Delta t) - \Delta SI(t) = \frac{D}{\lambda(x, y, z)^2} \nabla^2 (\Delta SI) + \frac{Q}{\alpha} k - k' \Delta SI(t) + k'b \qquad (11),$$

wherein, $\Delta t$ is the sampling time in each unit,

$$\nabla^2 (\Delta SI) = \sum_{i=x,y,z} \Delta SI(i + \Delta i) + \Delta SI(i - \Delta i) - 2\Delta SI(i) \qquad ,$$

$\Delta i$ represents the space distance of sampling along the axis i. It depends on the space resolution of the image along the axis i.

For the discrete equation (11), the space tortuosity $\lambda$, the loss ratio k' and the part volume ratio $\alpha$ can be fit by the least squares method, i.e. the solution of the below optimization problem is required:

$$\min f(\lambda, \alpha, k') = \sum_x \sum_y \sum_z \sum_t \left( \frac{D}{\lambda(x, y, z)^2} \frac{\partial^2 (\Delta SI)}{\partial r^2} + \frac{Q}{\alpha} k - k'(\Delta SI) + k'b - \Delta SI(t + \Delta t) + \Delta SI(t) \right)^2 \qquad (12).$$

Write the equation (12) in a matrix form and get:

$$\min f(\lambda, \alpha, k') = \|Ax - b\|_2^2 \qquad (13),$$

wherein, the nonvanishing element in the line (x, y, z, t,:) of the matrix A is :

$$\frac{\partial^2 \Delta SI}{\partial^2 r} + k - \Delta SI + b \qquad (14),$$

the line (x, y, Z, t,) of the known vector b is:

$$b(x, y, z, t,:) = \Delta SI(t + \Delta t) - \Delta SI(t) \qquad (15),$$

the substitution variable x is:

$$x = [\frac{D}{\lambda(x, y, z)^2}, \frac{Q}{\alpha}, k']^T \qquad (16).$$

When the time sampling rate and the space sampling rate are high, the discrete equation (11) can be equivalent to the continuity equation (10). Under normal circumstances, the real sampling rate is low, so the obtained space tortuosity $\lambda$, the loss ratio k' and the part volume rate $\alpha$ are not strictly the values but the values of "average" concept.

The molecular diffusion from a point in ECS may have anisotropic feature, i.e. the molecular may quickly spread in one direction but be blocked in another direction. Therefore, we need to consider the anisotropy of molecular diffusion. Assuming the main direction of the anisotropic diffusion tensor is consistent with coordinate axis, the equation (2) can be generalized to the anisotropic case:

$$\frac{\partial C}{\partial t} = \frac{D}{\lambda_x^2}\frac{\partial^2 C}{\partial x^2} + \frac{D}{\lambda_y^2}\frac{\partial^2 C}{\partial y^2} + \frac{D}{\lambda_z^2}\frac{\partial^2 C}{\partial z^2} + \frac{Q}{\alpha} - k'C \tag{17},$$

Sykova E and Nicholson C have solved the equation (3):

$$C = \frac{Q\lambda_x\lambda_y\lambda_z}{8\pi D\alpha R}[erfc(\frac{R}{2\sqrt{Dt}} + \sqrt{k't})\exp(R\sqrt{\frac{k'}{D}}) + erfc(\frac{R}{2\sqrt{Dt}} - \sqrt{k't})\exp(-R\sqrt{\frac{k'}{D}})] \tag{18},$$

wherein, $R = \sqrt{\lambda_x^2 x^2 + \lambda_y^2 y^2 + \lambda_z^2 z^2}$

In an isotropic medium, $\lambda = \lambda_x = \lambda_y = \lambda_z$, the equation (18) reduced to (17).

It can be seen in the equation (17) that the tortuosity is a constant which can not reflect the actual ECS structure. In order to reflect the real structure of ECS, the discrete equation (11) can be generalized to the anisotropic case:

$$C(t+\Delta t) - C(t) = \sum_{i=x,y,z}\frac{D}{\lambda_i(x,y,z)^2}\nabla^2 C_i + \frac{Q}{\alpha} - k'C(t) \tag{19}.$$

Similarly, the space tortuosity $\lambda$, the loss rate k' and the part volume ratio a along the three axial are to be solved using the method of least squares. Different from the isotropic case, the coefficient matrix A and substitution variable x are changed into:

$$A(x,y,z,t,:) = [\nabla^2 C_x, \nabla^2 C_y, \nabla^2 C_z, 1, -C(t)] \tag{20},$$

$$x = [\frac{D}{\lambda_x(x,y,z)^2}, \frac{D}{\lambda_y(x,y,z)^2}, \frac{D}{\lambda_z(x,y,z)^2}, \frac{Q}{\alpha}, k']^T \tag{21}.$$

The undetermined parameters of equation (19) are almost 2 times larger than that of the isotropic discrete equations. The equation (19) has a higher precision, i.e. the objective function equation (12) is smaller so that the real diffusion concentration can be better predicted.

Large numbers of the tracer concentration values, the corresponding position parameters and the corresponding time parameters should be imported into the equation for fitting to solve the flow parameters of brain interstitial fluid. According to the method of the present invention, using the concentration value and its relevant position parameter and time parameters of each pixel in concentration distribution map which is automatically read by corresponding computer program, the flow parameter of brain interstitial fluid can be automatically fit.

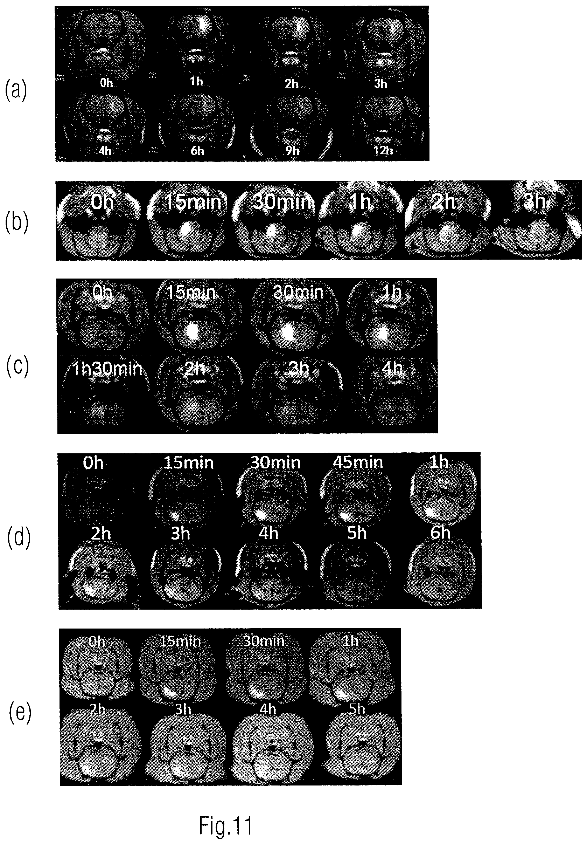

FIG. 11 shows the images scanned in different time of SD rat after anesthesia in magnetic resonance imaging wrist coil, after injection of Gd-DTPA (2μL,10mM) by microinjection via different injection site. The injection site located in (a)the caudate nucleus, (b) located in the substantia nigra striatum, (c) located in the thalamus, (d) located in the occipital cortex and (e) located in the subcortical white matter.

Via calculating the collected images from regions of the caudate nucleus, the thalamus, the substantia nigra and the striatum, the pillow image cortex and the brain subcortical white matter of SD rats by the simulation method of the present invention, the diffusion parameters in different regions in the brains can be obtained. The results are shown in Table 1. Table 2 shows anisotropy of the diffusion parameters for the caudate nucleus of the rats. Table 3 shows the distribution volume changing with time of the tracer Gd-DTPA after injection through caudate nucleus in the brain.

Table1: diffusion parameters in different regions of SD rats' brain

| Brain area | D* ($\times 10^{-4}$/mm$^2 \cdot$s$^{-1}$) | k' ($\times 10^{-4}$/mM$\cdot$ L$^{-1}\cdot$ s$^{-1}$) | λ |
|---|---|---|---|
| caudate putamen (CPu) | 3.38 | 0.76 | 1.24 |
| substantianigra (SN) | 2.06 | 8.28 | 1.59 |
| thalamus (T) | 3.28 | 1.44 | 1.26 |
| occipital cortex (Oc) | 3.25 | 1.55 | 1.26 |
| medial prefrontal cortex (mPFC) | 2.31 | 2.38 | 1.50 |

Table 2: the anisotropic diffusion parameters of rats' caudate nucleus regions

| parameters | | D* ($\times 10^{-4}$/mm$^2 \cdot$s$^{-1}$) | k' ($\times 10^{-5}$/mM$\cdot$L$^{-1}\cdot$s$^{-1}$) |
|---|---|---|---|
| | -X | 3.57 ± 0.88 | 7.14 ± 3.06 |
| | X | 3.73 ± 1.10 | 7.67 ± 3.96 |
| direction | -Y | 3.05 ± 0.93 | 7.59 ± 4.04 |
| | Z | 2.54 ± 1.27 | 7.74 ± 4.07 |
| | -Z | 4.01 ± 0.60 | 7.88 ± 6.24 |

Table 3: The change rule of distribution volume with Gd-DTPA after injection in caudate nucleus

| Time (h) | Distribution Volume(Vd/mm$^3$) | Distribution Volume Fraction (%) |
|---|---|---|
| 1 | 724.75 | 7.55 |
| 2 | 910.50 | 9.48 |
| 3 | 907.25 | 9.45 |
| 4 | 787.57 | 8.20 |
| 6 | 668.50 | 6.96 |
| 9 | 411.57 | 4.29 |
| 12 | 128.13 | 1.33 |

Calculation method about distribution volume (Vd), distribution volume fraction and general clearance of the tracer in the living organism is as follows: reconstructing the registered and subtracted images into axial images; quantitative measuring the signal increment region in image of each layer via the Matlab program as shown in figures; and recording the number of pixels in the increment region and the signal intensity value of each pixel. According to the integral principle, we can obtain the distribution volumes (Vd) of the tracer (Gd-DTPA) from different times in the brain hemisphere ECS via adding up the increment region of each layer. The distribution volume fraction of Gd-DTPA in the brain hemisphere ECS can be obtained by dividing the Vd from different times by the volume of the brain hemisphere of the rat. The macro clearance is defined as the total signal intensity at a previous time subtracting the total signal intensity at a later time, then dividing the initial total signal intensity.

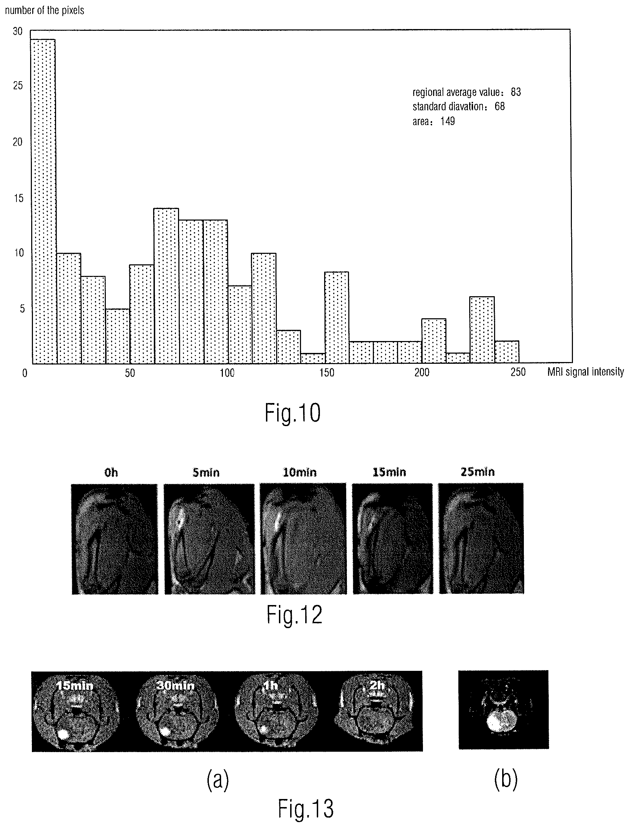

**FIG. 9** shows using Matlab software to measure the un-increment region subtracting increment region. The selected region in the FIG. is obtained by subtracting the image before increment from the increment image. The increment region is shown as the "bright region" and the other un-increment region is shown as the "dark region". **FIG. 10** shows the graphical results of the increment region in **FIG. 9,** wherein the abscissa is the signal intensity and the ordinate is the number of pixels. Using the bulleted method above can measure the Vd, the distribution volume fraction and the macro clearance of the caudate nucleus. Table 4 shows the macro clearances from different times after the Gd-DTPA injection to the caudate nucleus of eight SD rats

Table 4: the macro clearances from different times after the Gd-DTPA injection to the caudate nucleus of eight SD rats

| time(h) | Macro clearance(/h) |
|---|---|
| 1-2 | 0.23 |
| 2-3 | 0.12 |
| 3-4 | 0.19 |
| 4-6 | 0.11 |
| 6-9 | 0.06 |
| 9-12 | 0.08 |

Although the above invention is for the brain ECS of the SD rat, the present invention is applicable to other the living organisms. **FIG. 12** shows the diffusion MRI images scanned in different time after injecting the Gd-DTPA into the skeletal muscle of the rats. The left high signal region adjacent to the femur is the Gd-DTPA's diffusion region after injection.

Table 5 shows the diffusion parameters after Gd-DTPA injection via the skeletal muscle gap. Table 6 shows the time-varying changing rule of the distribution volume of the Gd-DTPA in the skeletal muscle gap of the rat. Table 7 shows the macro clearance of the Gd-DTPA in the skeletal muscle gap

Table 5: the diffusion parameters of the Gd-DTPA in the skeletal muscle gap

| parameters | | $D^*$ ($\times 10^{-4}$/mm$^2 \cdot$s$^{-1}$) | k' ($\times 10^{-4}$/mM$\cdot$L$^{-1} \cdot$s$^{-1}$) |
|---|---|---|---|
| | -X | 2.611 | 2.359 |
| | X | 2.930 | 2.870 |
| direction | -Y | 8.235 | 10.940 |
| | Z | 9.537 | 7.817 |
| | -Z | 7.154 | 5.083 |

Table 6: the time-varying change rule of distribution volume of the Gd-DTPA in the skeletal muscle gap

| time(min) | Vd(mm$^3$) |
|---|---|
| 5 | 407 |
| 10 | 383 |
| 15 | 234 |
| 25 | 0 |

Table 7: the macro clearance the Gd-DTPA in the skeletal muscle gap of the rat

| time(h) | Macro clearance(/h) |
|---|---|
| 5-10 | 0.28 |
| 10-15 | 0.36 |
| 15-25 | 0.36 |

**FIG. 13** shows the diffusion images of Gd-DTPA in the glioma model of the brain of the rat, wherein (a) the high signal areas of the T1WI are the diffusion areas of Gd-DTPA and the low signal areas are the gliomas locations, (b) the high signal area of the T2WI is the gliomas location..

Table 8 shows the diffusion parameter of Gd-DTPA in the glioma brain of the rat.

Table 8: the diffusion parameter of Gd-DTPA in the glioma brain of the rat

| parameters | | $D^*(\times 10^{-4}/\text{mm}\cdot\text{s}^{-1})$ | k' $(\times 10^{-4}/\text{mM}\cdot\text{L}^{-1}\cdot\text{s}^{-1})$ |
|---|---|---|---|
| direction | -X | 4.02 | 3.31 |
| | X | 3.60 | 3.13 |
| | -Y | 4.82 | 3.12 |
| | Z | 4.61 | 3.15 |
| | -Z | 5.63 | 3.05 |

Using the same method as used in the brain ECS, we can learn the time-varying change rule of distribution volume of the Gd-DTPA in the glioma brain of the rat. The results are in Table 9 and Table 10.

Table 9: the time-varying change rule of distribution volume of the Gd-DTPA in the glioma brain of the rat

| Time (h) | Vd(mm$^3$) | Distribution Volume Fraction (%) |
|---|---|---|
| 0.25 | 466 | 4.85 |
| 0.5 | 651 | 6.78 |
| 1 | 720 | 7.50 |
| 2 | 503 | 5.24 |
| 3 | 0 | 0 |

Table 10: the macro clearances of the Gd-DTPA in the glioma brain of the rat

| Time (h) | Macro clearances (/h) |
|---|---|
| 0.5-1 | 0.23 |
| 1-2 | 0.36 |
| 2-3 | 0.41 |

The calculation method provided in the present invention can use the MRI scan image of the interested the living organism to accurately calculate the tracer distribution in the gap of the living organism and the diffusion parameter in the ECS, which is helpful to the research of diseases diagnosis and pharmacokinetics.

The invention has been described with reference to the preferred embodiments. Modifications and alterations may occur to others upon reading and understanding the preceding detailed description. It is intended that the invention be construed as including all such modifications and alterations insofar as they come within the scope of the appended claims.

**Claims**

1. A method for computing parameters of a tracer distributing in a living organism and diffusing in an Extracellular Space, ECS, comprising:

(1) measuring a magnetic resonance signal increment $\Delta SI$ corresponding to different tracer concentrations C in agarose;
(2) according to the magnetic resonance signal increments measured in the step (1), determining an upper limit of the tracer concentration C up to which the tracer concentration C has a linear relation with the magnetic resonance signal increment $\Delta SI$ and determining the linear relation between the magnetic resonance signal increment $\Delta SI$ and the tracer concentration C;
(3) obtaining magnetic resonance images from different times of the living organism, who has been injected the tracer with different concentrations of tracer at injection sites Q;
(4) registering regions of interest from the obtained magnetic resonance images to obtain registered images; and

(5) fitting parameters of the tracer distributing in the living organism according to the registered images, which comprises:

(5.1) determining a diffusion equation of the tracer, wherein fitting parameters of the tracer distributing in the living organism according to the registered images further comprises:

(5.2) converting the diffusion equation determined in the step (5.1) according to the linear relation between the tracer concentration and the magnetic resonance signal increment, and

(5.3) solving the diffusion equation acquired in the step (5.2) to fit diffusion parameters including diffusion coefficient D*, loss ratio k' and part volume ratio $\alpha$ of the tracer, wherein the part volume ratio $\alpha$ is a ratio of the volume of the Extracellular Space of a brain of the living organism to the volume of the whole brain, and the loss ratio k' is a clearance rate of the tracer in the brain which is a constant,

wherein the diffusion equation of the tracer in the standard coordinates obtained in the step (5.1) is:

$$\frac{\partial C}{\partial t} = \frac{D}{\lambda(x,y,z)^2}\nabla^2 C + \frac{Q}{\alpha} - k'C$$ .

wherein D is a free diffusion coefficient of the tracer,
$\lambda(x, y, z)$ is a tortuosity of a point (x, y, z) in the living organism,
C is the tracer concentration which is obtained via the magnetic resonance signal increment $\Delta SI$,
t is a time interval between tracer injection and magnetic resonance image acquisition;

the diffusion equation in an anisotropic condition can be equivalently transformed into :

$$\frac{\partial C}{\partial t} = \frac{D}{\lambda_x^2}\frac{\partial^2 C}{\partial x^2} + \frac{D}{\lambda_y^2}\frac{\partial^2 C}{\partial y^2} + \frac{D}{\lambda_z^2}\frac{\partial^2 C}{\partial z^2} + \frac{Q}{\alpha} - k'C$$ .

wherein $\lambda_x$, $\lambda_y$ and $\lambda_z$ are the tortuosities of the living organism along the axis X,Y and Z respectively, the equation after converting in the step (5.2) is:

$$\frac{\partial(\Delta SI)}{\partial t} = \frac{D}{\lambda(x,y,z)^2}\frac{\partial^2(\Delta SI)}{\partial r^2} + \frac{Q}{\alpha}k - k'(\Delta SI) + k'b$$ -

wherein k and b are constants,
$\Delta SI$ is the magnetic resonance signal increment,
D is the real space distance corresponding to a single pixel;

the equation above is converted into a discrete model in the step (5.3) as follows:

$$\Delta SI(t+\Delta t) - \Delta SI(t) = \frac{D}{\lambda(x,y,z)^2}\nabla^2(\Delta SI) + \frac{Q}{\alpha}k - k'\Delta SI(t) + k'b$$ -

wherein $\Delta t$ is the sampling time in each unit,
$\Delta l$ is the space distance of the sample in any direction (axis i);

wherein the equation obtained in the step (5.3) can be fitted via the least squares method using the following equation:

$$\min f(\lambda,\alpha,k') = \sum_x\sum_y\sum_z\sum_t\left(\frac{D}{\lambda(x,y,z)^2}\frac{\partial^2(\Delta SI)}{\partial r^2} + \frac{Q}{\alpha}k - k'(\Delta SI) + k'b - \Delta SI(t+\Delta t) + \Delta SI(t)\right)^2$$

wherein min $f(\lambda,\alpha,k')$ is the minimum value of the variance, the above equation is expressed in matrix form as:

$$\min f(\lambda,\alpha,k') = \|Ax - b\|^2.$$

the non-zero element in the row (x, y, Z, t,:) in the matrix A is:

$$\frac{\partial^2 \Delta SI}{\partial^2 r} + k - \Delta SI + b,$$

the row (x, y, Z, t,:) of known vector b is:

$$b(x,y,z,t,:) = \Delta SI(t+\Delta t) - \Delta SI(t)$$

the substitution variable x is:

$$x = \left[\frac{D}{\lambda(x,y,z)^2}, \frac{Q}{\alpha}, k'\right]^T.$$

2. The method according to claim 1, wherein the image registration steps comprises :

$$
\begin{aligned}
I(f,g) &= H(f) + H(g) - H(f,g) \\
&= -\sum_{i=1}^{N} p(i)\log(p(i)) - \sum_{j=1}^{M} p(j)\log(p(j)) + \sum_{i=1}^{N}\sum_{j=1}^{M} p(i,j)\log(p(i)) \\
&= \sum_{i=1}^{N}\sum_{j=1}^{M} p(i,j)\log(\frac{p(i,j)}{p(i)p(j)})
\end{aligned}
$$

wherein, $H(f)$, H(g) and H(f,g) are the edge entropy and joint entropy of the two images,
N and M are the numbers of grayscale samples of the two images,
p(i), p(j) and p(i, j) are the possibilities of the grayscale appearing,
f and g are the two images that are to be registered, and
I (f, g) represents the mutual information defined as the difference between edge entropy and joint entropy of the two images.

3. The method according to claim 1, wherein the registration process of the step (4) further comprises a grayscale correction:

$$I(x,y,z) = f_{MR}(x,y,z)B(x,y,z) + n(x,y,z),$$

wherein, $f_{MR}$ is the real NMR signals not polluted by noise and bias field,
I is the imaging signal,
B is the grayscale bias field, and
n is the artificial noise introduced in the imaging process.

4. The method according to claim 3, wherein the grayscale correction further comprises:

$$g' = u_2 + (g - u_1) \times \frac{\sigma_2}{\sigma_1},$$

wherein, (u1,σ1) and (u2 ,σ2) are the mean values and the variances of the magnetic resonance signal intensities in two areas to be corrected,
g is the real MRI image signal intensity,
g' is the corrected MRI image signal intensity, and
the two areas to be corrected are picked from the same positions in imaging regions from a uniform tissue.

**5.** The method according to claim 1, wherein the tracer is Gd-DTPA, the diffusion parameter of the living organism comprises the effective diffusion efficient D*, the tortuosity λ and the clearance rate k' of Gd-DTPA in the Extracellular Space.

## Patentansprüche

**1.** Verfahren zum Berechnen von Parametern eines Tracers, der sich in einem lebenden Organismus verteilt und in einem Extrazellularraum, ECS, diffundiert, umfassend:

(1) Messen eines Magnetresonanzsignalinkrements ΔSI, das verschiedenen Tracerkonzentrationen C in Agarose entspricht;
(2) entsprechend den im Schritt (1) gemessenen Magnetresonanzsignalinkrementen, Bestimmen einer oberen Grenze der Tracerkonzentration C, bis zu der die Tracerkonzentration C eine lineare Beziehung mit dem Magnetresonanzsignalinkrement ΔSI hat, und Bestimmen der linearen Beziehung zwischen dem Magnetresonanzsignalinkrement ΔSI und der Tracerkonzentration C;
(3) Erlangen von Magnetresonanzbildern aus verschiedenen Zeiten des lebenden Organismus, dem der Tracer mit unterschiedlichen Konzentrationen an den Injektionsstellen Q injiziert wurde;
(4) Registrieren von Regionen von Interesse aus den erhaltenen Magnetresonanzbildern, um registrierte Bilder zu erhalten; und
(5) Anpassen von Parametern des im lebenden Organismus verteilten Tracers gemäß den registrierten Bildern, was umfasst:
(5.1) Bestimmen einer Diffusionsgleichung des Tracers,
wobei
Anpassen von Parametern des im lebenden Organismus verteilten Tracers gemäß den registrierten Bildern ferner umfasst:

(5.2) Umwandeln der im Schritt (5.1) bestimmten Diffusionsgleichung gemäß der linearen Beziehung zwischen der Tracerkonzentration und dem Magnetresonanzsignalinkrement, und
(5.3) Lösen der im Schritt (5.2) ermittelten Diffusionsgleichung, um Diffusionsparameter einschließlich des Diffusionskoeffizienten D*, des Verlustverhältnisses k' und des Teilvolumenverhältnisses α des Tracers anzupassen, wobei das Teilvolumenverhältnis α ein Verhältnis des Volumens des Extrazellularraums eines Gehirns des lebenden Organismus zum Volumen des gesamten Gehirns ist und das Verlustverhältnis k' eine Clearance-Rate des Tracers im Gehirn ist, die eine Konstante ist,

wobei die Diffusionsgleichung des Tracers in den im Schritt (5.1) erhaltenen Standardkoordinaten lautet:

$$\frac{\partial C}{\partial t} = \frac{D}{\lambda(x,y,z)^2} \nabla^2 C + \frac{Q}{\alpha} - k'C \quad,$$

wobei D ein freier Diffusionskoeffizient des Tracers ist,
λ (x, y, z) eine Tortuosität eines Punktes (x, y, z) im lebenden Organismus ist,
C die Tracerkonzentration ist, die über das Magnetresonanzsignalinkrement ΔSI erhalten wird,
t ein Zeitintervall zwischen Tracerinjektion und Magnetresonanzbilderlangung ist;

die Diffusionsgleichung in einer anisotropen Bedingung äquivalent transformiert werden kann in :

$$\frac{\partial C}{\partial t} = \frac{D}{\lambda_x^2} \frac{\partial^2 C}{\partial x^2} + \frac{D}{\lambda_y^2} \frac{\partial^2 C}{\partial y^2} + \frac{D}{\lambda_z^2} \frac{\partial^2 C}{\partial z^2} + \frac{Q}{\alpha} - k'C \quad,$$

wobei λx, λy und λz die Tortuositäten des lebenden Organismus entlang der Achsen X, Y bzw. Z sind, die Gleichung nach der Konvertierung im Schritt (5.2) lautet:

$$\frac{\partial\left(\Delta SI\right)}{\partial t} = \frac{D}{\lambda\left(x,y,z\right)^2}\frac{\partial^2\left(\Delta SI\right)}{\partial r^2}+\frac{Q}{\alpha}k-k'\left(\Delta SI\right)+k'b$$ ,

wobei k und b Konstanten sind,
ΔSI das Magnetresonanzsignalinkrement ist,
D der reale Raumabstand ist, der einem einzelnen Pixel entspricht;

die obige Gleichung im Schritt (5.3) wie folgt in ein diskretes Modell umgewandelt wird:

$$\Delta SI(t+\Delta t)-\Delta SI(t)=\frac{D}{\lambda(x,y,z)^2}\nabla^2\left(\Delta SI\right)+\frac{Q}{\alpha}k-k'\Delta SI(t)+k'b$$ ,

wobei Δt die Abtastzeit in jeder Einheit ist,
Δl der Raumabstand der Probe in jeder Richtung (Achse i) ist;

wobei die in dem Schritt (5.3) erhaltene Gleichung durch die Methode der kleinsten Quadrate unter Verwendung der folgenden Gleichung angepasst werden kann:

$$\min f\left(\lambda,\alpha,k'\right)=\sum_x\sum_y\sum_z\sum_t\left(\frac{D}{\lambda(x,y,z)^2}\frac{\partial^2\left(\Delta SI\right)}{\partial r^2}+\frac{Q}{\alpha}k-k'\left(\Delta SI\right)+k'b-\Delta SI\left(t+\Delta t\right)+\Delta SI\left(t\right)\right)^2$$ ,

wobei min $f\left(\lambda,\alpha,k'\right)$ der minimale Wert der Varianz ist,
die obige Gleichung in Matrixform ausgedrückt wird als

$$\min f\left(\lambda,\alpha,k'\right)=\left\|Ax-b\right\|^2$$ ,

das Nicht-Null-Element in der Zeile (x, y, Z, t,:) in der Matrix A ist:

$$\frac{\partial^2\Delta SI}{\partial^2 r}+k-\Delta SI+b$$ ,

die Zeile (x, y, Z, t,:) des bekannten Vektors b ist:

$$b\left(x,y,z,t,:\right)=\Delta SI\left(t+\Delta t\right)-\Delta SI\left(t\right)$$ ,

die Substitutionsvariable x ist:

$$x=\left[\frac{D}{\lambda\left(x,y,z\right)^2},\frac{Q}{\alpha},k'\right]^T$$ .

2.  Verfahren nach Anspruch 1, wobei die Bildregistrierungsschritte umfassen :

$$I(f,g) = H(f) + H(g) - H(f,g)$$

$$= -\sum_{i=1}^{N} p(i)\log(p(i)) - \sum_{j=1}^{M} p(j)\log(p(j)) + \sum_{i=1}^{N}\sum_{j=1}^{M} p(i,j)\log(p(i))$$

$$= \sum_{i=1}^{N}\sum_{j=1}^{M} p(i,j)\log(\frac{p(i,j)}{p(i)p(j)})$$

,

wobei $H(f)$, H(g) und H($f$,g) die Randentropie und die gemeinsame Entropie der beiden Bilder sind,

N und M die Anzahl der Graustufenproben der beiden Bilder sind,

p(i), p(j) und p(i, j) die Möglichkeiten des Auftretens der Graustufen sind,

f und g die beiden Bilder sind, die registriert werden sollen, und

I (f, g) die gegenseitige Information darstellt, die als Differenz zwischen Randentropie und gemeinsamer Entropie der beiden Bilder definiert ist.

3. Verfahren nach Anspruch 1, wobei der Registrierungsprozess des Schrittes (4) weiterhin eine Graustufenkorrektur umfasst:

$$I(x,y,z) = f_{MR}(x,y,z)B(x,y,z) + n(x,y,z)$$

,

wobei $f$MR die tatsächlichen NMR-Signale sind, die nicht durch Rauschen und Vorspannungsfeld verunreinigt sind,

I das bildgebende Signal ist,

B das Graustufen-Verzerrungsfeld ist, und

n das künstliche Rauschen ist, das in den Bildgebungsprozess eingebracht wird.

4. Verfahren nach Anspruch 3, wobei die Graustufenkorrektur weiter umfasst:

$$g' = u_2 + (g - u_1) \times \frac{\sigma_2}{\sigma_1}$$

,

wobei (u1, $\sigma$1) und (u2, $\sigma$2) die Mittelwerte und die Varianzen der Magnetresonanzsignalintensitäten in zwei zu korrigierenden Bereichen sind,

g die tatsächliche MRI-Bildsignalintensität ist,

g' die korrigierte MRI-Bildsignalintensität ist, und

die beiden zu korrigierenden Bereiche von den gleichen Positionen in bildgebenden Regionen aus einem einheitlichen Gewebe entnommen werden.

5. Verfahren nach Anspruch 1, wobei der Tracer Gd-DTPA ist, der Diffusionsparameter des lebenden Organismus die effektive Diffusionseffizienz D*, die Tortuosität $\lambda$ und die Clearance-Rate k' von Gd-DTPA im Extrazellularraum umfasst.

**Revendications**

1. Procédé de calcul de paramètres d'une distribution de traceur dans un organisme vivant et d'une diffusion dans un espace extracellulaire, ECS, consistant à :

(1) mesurer un incrément de signal de résonance magnétique ($\Delta$SI) correspondant à différentes concentrations en traceur (C) dans l'agarose ;

(2) selon les incréments de signal de résonance magnétique mesurés dans l'étape (1), déterminer une limite supérieure de la concentration en traceur (C) à laquelle la concentration en traceur (C) a une relation linéaire avec l'incrément de signal de résonance magnétique ($\Delta$SI) et déterminer la relation linéaire entre l'incrément de signal de résonance magnétique (ΔSI) et la concentration en traceur (C) ;

(3) obtenir des images de résonance magnétique à différents moments de l'organisme vivant, à qui a été injecté le traceur à différentes concentrations en traceur dans des sites d'injection (Q) ;

(4) enregistrer des régions d'intérêt à partir des images de résonance magnétique pour obtenir des images enregistrées ; et

(5) adapter les paramètres de la distribution de traceur dans l'organisme vivant selon les images enregistrées, qui consiste à :

(5.1) déterminer une équation de diffusion du traceur,

l'adaptation des paramètres de la distribution de traceur dans l'organisme vivant selon les images enregistrées consistant en outre à :

(5.2) convertir l'équation de diffusion déterminée dans l'étape (5.1) selon la relation linaire entre la concentration en traceur et l'incrément de signal de résonance magnétique, et

(5.3) résoudre l'équation de diffusion acquise dans l'étape (5.2) pour adapter les paramètres de diffusion y compris le coefficient de diffusion D*, le coefficient de perte k' et/ou le rapport de partie en volume $\alpha$ du traceur, le rapport de partie en volume $\alpha$ étant un rapport entre le volume de l'espace extracellulaire d'un cerveau de l'organisme vivant et le volume du cerveau entier, et le coefficient de perte k' étant une vitesse d'élimination du traceur dans le cerveau qui est une constante,

l'équation de diffusion du traceur dans les coordonnées standard obtenues dans l'étape (5.1) est :

$$\frac{\partial C}{\partial t} = \frac{D}{\lambda(x,y,z)^2}\nabla^2 C + \frac{Q}{\alpha} - k'C \quad ,$$

D étant un coefficient de diffusion libre du traceur,

$\lambda$ (x, y, z) étant une tortuosité d'un point (x, y, z) dans l'organisme vivant,

C étant la concentration en traceur qui est obtenue par l'incrément de signal de résonance magnétique (ΔSI),

t étant un intervalle temporel entre l'injection de traceur et l'acquisition d'image par résonance magnétique,

l'équation de diffusion dans une condition anisotrope pouvant être transformée de manière équivalente en :

$$\frac{\partial C}{\partial t} = \frac{D}{\lambda_x^2}\frac{\partial^2 C}{\partial x^2} + \frac{D}{\lambda_y^2}\frac{\partial^2 C}{\partial y^2} + \frac{D}{\lambda_z^2}\frac{\partial^2 C}{\partial z^2} + \frac{Q}{\alpha} - k'C \quad ,$$

$\lambda_x$, $\lambda_y$ et $\lambda_z$ étant les tortuosités de l'organisme vivant le long de l'axe X, Y et Z respectivement,

l'équation après la conversion dans l'étape (5.2) étant :

$$\frac{\partial(\Delta SI)}{\partial t} = \frac{D}{\lambda(x,y,z)^2}\frac{\partial^2(\Delta SI)}{\partial r^2} + \frac{Q}{\alpha}k - k'(\Delta SI) + k'b \quad ,$$

k et b étant des constantes,

ΔSI étant l'incrément de signal de résonance magnétique,

D étant la distance de l'espace réel correspondant à un seul pixel ;

l'équation ci-dessus étant convertie en un modèle discret dans l'étape (5.3) comme suit :

$$\Delta SI(t+\Delta t) - \Delta SI(t) = \frac{D}{\lambda(x,y,z)^2}\nabla^2(\Delta SI) + \frac{Q}{\alpha}k - k'\Delta SI(t) + k'b \quad ,$$

Δt étant le temps d'échantillonnage dans chaque unité,

Δl étant la distance d'espace de l'échantillon dans une quelconque direction (axe i) ;

l'équation obtenue dans l'étape (5.3) peut être adaptée par la méthode des moindres carrés à l'aide de l'équation suivante :

$$\min f\left(\lambda,\alpha,k'\right) = \sum_{x}\sum_{y}\sum_{z}\sum_{t}\left(\frac{D}{\lambda\left(x,y,z\right)^2}\frac{\partial^2\left(\Delta SI\right)}{\partial r^2} + \frac{Q}{\alpha}k - k'\left(\Delta SI\right) + k'b - \Delta SI\left(t+\Delta t\right) + \Delta SI\left(t\right)\right)^2,$$

$\min f\left(\lambda,\alpha,k'\right)$ étant la valeur minimum de l'écart,
l'équation ci-dessus étant exprimée dans la forme matricielle :

$$\min f\left(\lambda,\alpha,k'\right) = \left\|Ax - b\right\|^2,$$

l'élément non zéro dans la rangée (x, y, Z, t,:) dans la matrice A étant :

$$\frac{\partial^2 \Delta SI}{\partial^2 r} + k - \Delta SI + b,$$

la rangée (x, y, Z, t,:) du vecteur connu b étant :

$$b\left(x,y,z,t,:\right) = \Delta SI\left(t+\Delta t\right) - \Delta SI\left(t\right),$$

la variable de substitution x étant :

$$x = \left[\frac{D}{\lambda\left(x,y,z\right)^2}, \frac{Q}{\alpha}, k'\right]^T.$$

2. Procédé selon la revendication 1, l'étape d'enregistrement d'image comprend :

$$\begin{aligned}I(f,g) &= H(f) + H(g) - H(f,g)\\ &= -\sum_{i=1}^{N}p(i)\log(p(i)) - \sum_{j=1}^{M}p(j)\log(p(j)) + \sum_{i=1}^{N}\sum_{j=1}^{M}p(i,j)\log(p(i))\\ &= \sum_{i=1}^{N}\sum_{j=1}^{M}p(i,j)\log(\frac{p(i,j)}{p(i)p(j)})\end{aligned},$$

H(*f*), H(g) et H(*f*,g) étant l'entropie d'arête et l'entropie jointe des deux images,
N et M étant les nombres d'échantillons de gamme de gris des deux images,
p(i), p(j) et p(i, j) étant les possibilités d'apparition de gamme de gris,
f et g étant les deux images qui doivent être enregistrées, et
I (f, g) représentant les informations réciproques définies comme la différence entre l'entropie d'arête et l'entropie jointe des deux images.

3. Procédé selon la revendication 1, le processus d'enregistrement de l'étape (4) comprenant en outre une correction de la gamme de gris :

$$I(x,y,z) = f_{MR}(x,y,z)B(x,y,z) + n(x,y,z),$$

*f*MR étant les signaux réels NMR non pollués par le champ de bruit et de biais,

I étant le signal d'imagerie,

B étant le champ de biais de la gamme de gris, et

n étant le bruit artificiel introduit dans le processus d'imagerie.

4. Procédé selon la revendication 3, la correction de la gamme de gris comprenant en outre :

$$g^{'} = u_2 + (g - u_1) \times \frac{\sigma_2}{\sigma_1}$$

,

(u1,σ1) et (u2,σ2) étant des valeurs moyennes et les écarts des intensités de signal de résonance magnétique dans deux zones à corriger,

g étant l'intensité de signal d'image réelle IRM,

g' étant l'intensité de signal d'image IRM corrigée, et

les deux zones à corriger sont choisies dans les positions identiques dans des régions d'imagerie à partir d'un tissu uniforme.

5. Procédé selon la revendication 1, le traceur étant Gd-DTPA, le paramètre de diffusion de l'organisme vivant comprenant le coefficient de diffusion effectif D*, la tortuosité λ et la vitesse d'élimination k' de Gd-DTPA dans l'espace extracellulaire.

Fig.1

Fig.2

Fig.3

Fig.4

Fig.5

FIG.6

Fig.7

Fig.8

Fig.9

Fig.11

EP 2 803 317 B1

number of the pixels

regional average value: 83
standard diavation: 68
area: 149

MRI signal intensity

Fig.10

0h    5min    10min    15min    25min

Fig.12

(a)                    (b)

Fig.13

29

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2010102086244

- WO 2011069283 A1

### Non-patent literature cited in the description

- **M. PELLERIN.** Incorporating Contrast Agents Diffusion Into the Analysis of DCE-MRI Data. *Magnetic Resonance in Medicine,* 2007, vol. 58, 1124-1134